# EUROPÄISCHE PATENTSCHRIFT

(11) **EP 0 627 957 B1**
(45) Veröffentlichungstag und Bekanntmachung des Hinweises auf die Patenterteilung: **22.01.1997**
(21) Anmeldenummer: 93903998.8
(22) Anmeldetag: 19.02.1993
(51) Int. Cl.: B01J 2/28, B01J 8/14, B01J 8/18, B01J 20/00, B01D 53/02, B01D 53/14, C02F 1/28, C09K 3/32

(54) **VERFAHREN ZUR REINIGUNG VON TRINKWASSER ODER ABWASSER UND/ODER ABGAS**
PROCESS FOR PURIFICATION OF DRINKING WATER OR WASTE WATER AND/OR GAS
PROCEDE POUR PURIFICATION D'EAU POTABLE OU D'EAUX USEES ET/OU DE GAZ D'ECHAPPEMENT

(30) Priorität: 24.02.1992 DE 4205572
(43) Veröffentlichungstag der Anmeldung: 14.12.1994
(73) Patentinhaber: Linde Aktiengesellschaft, 65189 Wiesbaden (DE)
(72) Erfinder: FUCHS, Uwe, D-80689 München (DE)
(74) Vertreter: Kasseckert, Rainer
(86) Internationale Anmeldenummer: EP9300403
(87) Internationale Veröffentlichungsnummer: WO9316792

(56) Entgegenhaltungen:
- US-A- 3 998 988
- US-A- 4 759 922
- US-A- 5 009 790

## Beschreibung

Die Erfindung betrifft ein Verfahren zur Reinigung von Trinkwasser oder Abwasser und/oder Abgas, wobei das Trinkwasser oder Abwasser und/oder Abgas mit Reaktionsteilchen in Kontakt gebracht wird, die die Reinigung fördern oder ermöglichen.

In der Verfahrenstechnik werden vielfach chemische, physikalische oder biologische Reaktionen in Gegenwart von Reaktionsteilchen durchgeführt, die entweder katalytisch wirken, d.h. eine bestimmte Reaktion zwischen den Reaktanten fördern, oder selbst als Reaktant wirken, d.h. selbst eine Reaktion mit einem anderen Reaktanten eingehen. So werden beispielsweise in der Abwassertechnik Trägerteilchen als Aufwuchsfläche für Mikroorganismen verwendet. Die mit Biomasse bewachsenen Trägerteilchen wirken als Biokatalysatoren und fördern den Abbau von Abwasserinhaltsstoffen. Es ist auch bekannt, die Trägerteilchen mit Aktivkohle zu dotieren, so daß die Trägerteilchen aufgrund der adsorptiven Wirkung der Aktivkohle selbst mit den Abwasserinhaltsstoffen physikalisch reagieren. Die Verwendung von Katalysatormaterialien oder Trägerteilchen für Biomasse ist auch aus der physikalischchemischen bzw. biologischen Abgasreinigung bekannt. Auch bei Fermentationsprozessen in der Biotechnik werden Trägerteilchen als Aufwuchsflächen für Biomasse eingesetzt.

In gängigen Wirbel- oder Schwebebettreaktoren zur Abwasser- oder Abgasreinigung werden vor allem Sand, Kies, Blähton, Kunststoffgranulate oder Schaumstoffwürfel als Trägerteilchen eingesetzt. Diese Trägerteilchen weisen alle eine räumliche Struktur auf und verursachen somit im Reaktor ein gewisses Totvolumen. Bei den porösen Trägern können darüberhinaus Stofftransportprobleme auftreten, so daß von vielen Gutachtern und Ingenieuren nur die äußere Trägeroberfläche als Aufwuchsfläche für die Biomasse berechnet wird. Außerdem weisen die bekannten Reaktionsteilchen nur eine geringe spezifische Oberfläche auf. Das spezifische Gewicht der Teilchen ist oft zu groß (z.B. bei Sand) oder zu klein (z.B. bei Styroporschaum). Die mechanische und/oder chemische Haltbarkeit läßt überdies oft zu wünschen übrig. Hohe Materialkosten lassen einen wirtschaftlichen Einsatz der Trägerteilchen oft nicht zu. Manche Materialien sind darüberhinaus z.B. für die Trinkwasseraufbereitung aus physiologischen Gründen nicht zugelassen.

Aufgabe der vorliegenden Erfindung ist es daher, ein wirtschaftliches Verfahren zur Reinigung von Trinkwasser oder Abwasser und/oder Abgas zur Verfügung zu stellen, das die beschriebenen Nachteile des Standes der Technik nicht aufweist.

Diese Aufgabe wird erfindungsgemäß dadurch gelöst, daß als Reaktionsteilchen im wesentlichen planare Teilchen mit einer Dicke von ca. 5 µm bis ca. 1500 µm und einer Fläche von ca. 5 (mm)² bis ca. 1000 (mm)² verwendet werden, die in einem Reaktor zum Schweben gebracht und/oder in Bewegung versetzt werden.

Bevorzugt werden Reaktionsteilchen mit einer Dicke von ca. 15 µm bis ca. 500 µm, besonders bevorzugt von ca. 25 µm bis ca. 150 µm eingesetzt.

Durch die planare Struktur wird eine hohe spezifische Besiedlungs- und/oder Reaktionsfläche erreicht, die darüberhinaus - im Gegensatz zu porösen Materialien - frei zugänglich ist. Das spezifische Gewicht dieser Konfetti-ähnlichen Teilchen ist je nach Material oder Materialkombination frei wählbar, so daß die Teilchen im Reaktor ohne großen Energieaufwand leicht fluidisierbar sind und sich, falls erforderlich, leicht pumpen lassen. Sollen die Reaktionsteilchen in einem mit Flüssigkeit gefüllten Reaktor eingesetzt werden, so wird vorzugsweise das spezifische Gewicht der Teilchen so eingestellt, daß es in etwa dem der Flüssigkeit entspricht. Beim Einsatz in Reaktoren zur Abwasserreinigung werden bevorzugt Reaktionsteilchen verwendet, deren spezifisches Gewicht ca. 0,85 bis ca. 0,95 beträgt. Nachdem sich diese Reaktionsteilchen während einer Anfahrphase mit Biomasse bewachsen haben, besitzen sie ein spezifisches Gewicht von etwa 1, so daß sie freibeweglich im Abwasser schweben und mit äußerst geringem Energieaufwand in Bewegung versetzt werden können, wodurch die Wirtschaftlichkeit der Abwasserreinigung erhöht wird. Bei Verwendung der Reaktionsteilchen in einem Verfahren zur biologischen Abwasserreinigung, biologischen Abgasreinigung, Fermentation oder allgemein einem Verfahren aus der Biotechnologie stellt sich ein sehr aktiver Biofilm auf den Reaktionsteilchen ein, der sich ständig verjüngt. Auf diese Weise kann die Reinigungsleistung bzw. Produktausbeute wesentlich gegenüber herkömmlichen Verfahren gesteigert werden.

Das erfindungsgemäße Verfahren eignet sich jedoch nicht nur für solche Zwecke, bei denen es auf einen biologischen Bewuchs der Reaktionsteilchen ankommt. Auch die Reaktionsteilchen als solche können zur Durchführung von Reaktionen eingesetzt werden. Dabei wird zweckmäßigerweise das Material der Reaktionsteilchen so ausgewählt, daß die gewünschten Reaktionen auf der Oberfläche der Reaktionsteilchen stattfinden. Die Reaktionsteilchen können hierbei als Katalysatoren wirken, die die Reaktionen zwischen den Reaktanten beschleunigen oder aber selbst mit den Reaktanten reagieren.

Beispielsweise können die Reaktionsteilchen aus Metallfolien bestehen, wobei die eingesetzten Metalle die gewünschten Reaktionen in Gang setzen. Ein Beispiel hierfür ist die Nitratentfernung aus Trinkwasser. Dabei wird das Trinkwasser z.B. in einem Reaktor mit den Reaktionsteilchen durchmischt. Die Reaktionsteilchen bestehen aus Aluminium-Folie-Stücken. Unter bestimmten Bedingungen reduziert das Leichtmetall das Nitrat vorwiegend zu gasförmigem Stickstoff. Am effektivsten ist die Reaktion bei pH-Werten zwischen 9,1 und 9,3.

Eine andere Möglichkeit besteht darin, Reaktionsteilchen zu verwenden, die mit reaktiven Stoffen dotiert sind. Beispiele hierfür sind die Dotierung mit Zeolithpulver (z.B. zur Ammonium-, Nitrat- oder CKW-Entfernung aus Grundwasser) oder mit Eisen (z.B. zur Rest-Phosphat-Entfernung). Auch die Dotierung mit Adsorptionsmitteln, wie Aktivkohle, bietet in bestimmten Anwendungsfällen Vorteile.

Gemäß einer besonders bevorzugten Ausführungsform der Erfindung werden im wesentlichen planare Reaktionsteilchen verwendet, deren Oberfläche zusätzlich strukturiert ist. Insbesondere sind solche Reaktionsteilchen vorteilhaft, die Löcher mit einem Durchmesser von ca. 10 µm bis ca. 1000 µm, vorzugsweise 200 bis 600 µm aufweisen. Die Lochdichte beträgt zweckmäßigerweise ca. 100 bis ca. 250 Löcher/cm². Derartige Reaktionsteilchen sind aus Kunststoff-Folien gefertigt, die in Stücken mit einer Fläche von jeweils ca. 5 (mm)² bis ca. 1000 (mm)² geschnitten sind.

Auch Prägungen der Reaktionsteilchen erweisen sich für die Effizienz des Verfahrens als vorteilhaft. Insbesondere werden Dellen mit einem Durchmesser von ca. 10 µm bis ca. 1000 µm und einer Tiefe von bis zu ca. 0,5 mm bevorzugt. Auch durch Ätzen oder Aufrauhen der Folienoberfläche können die gewünschten Reaktionsteilchen mit den günstigen Eigenschaften hergestellt werden.

Bei biologischen Verfahren, z.B. bei der Abwasser- oder Abgasreinigung oder bei Fermentationsprozessen, werden vorzugsweise Reaktionsteilchen aus Polymerverbindungen verwendet, die eine Oberflächenbehandlung erfahren haben. Insbesondere eignet sich hierfür die sogenannte Korona-Behandlung. Hierbei handelt es sich um eine elektrische Entladung, bei der Ozon freigesetzt wird, das wiederum die Matrix des Teilchenmaterials angreift und freie Valenzen schafft. Auf diese Weise werden die elektrischen Eigenschaften der Teilchenoberfläche verändert, so daß sich die Mikroorganismen leichter auf der Oberfläche festsetzen können.

Eine Weiterbildung des Verfahrens sieht vor, solche Reaktionsteilchen zu verwenden, auf deren Oberfläche Fasern z.B. durch Aufkaschieren bzw. Bevliesen aufgebracht wurden. Es kann auch ein Vlies aufgeklebt oder aufgeschweißt werden. Vorzugsweise werden Polyester- oder Polyolefinfädchen auf die Oberfläche aufgebracht. Dabei beträgt die Schichtdicke derart beschichteter Reaktionsteilchen maximal ca. 0,5 mm bis ca. 1 mm. Die Beschichtung kann ein- oder beidseitig erfolgen. Das Grundmaterial der verwendeten Reaktionsteilchen selbst besteht vorzugsweise aus Polyolefinen, insbesondere Polypropylen oder Polyethylen. Diese Materialien sind sehr billig und besitzen ein sehr günstiges spezifisches Gewicht von ca. 0,85 bis 0,95 g/cm³. Nach dem Bewachsen mit Biomasse ergibt sich ein spezifisches Gewicht um 1, so daß diese Reaktionsteilchen in wäßrigen Lösungen, z.B. im Abwasser im bewachsenen Zustand schweben. Es ist daher außerordentlich wenig Energie nötig, um die Reaktionsteilchen in Bewegung zu versetzen. Dies wirkt sich insbesondere auf die Wirtschaftlichkeit von Abwasserreinigungverfahren positiv aus. Auch Polystyrol eignet sich als Grundmaterial für die Reaktionsteilchen.

Gemäß einer Weiterbildung des Erfindungsgedankens werden Reaktionsteilchen aus Kunststoffgewebe, insbesondere thermofixiertem Kunststoffgewebe in gewirkter Form, verwendet. Diese Gewebe sind im wesentlichen planar, besitzen aber bereits eine gewisse Oberflächenstruktur, wodurch die gewünschten Reaktionen und das Aufwachsen von Mikroorganismen noch beschleunigt werden.

Das erfindungsgemäße Verfahren wird in Reaktoren durchgeführt. Insbesondere ist das Verfahren für die Abwasser- und/oder Abgasreinigung vorgesehen. Hierzu werden die Reaktionsteilchen in dem Reaktor mit dem Abwasser und/oder Abgas in Kontakt gebracht. Eine Variante der Erfindung sieht vor, das Abwasser und/oder Abgas durch ein fluidisiertes Bett der Reaktionsteilchen hindurch zu leiten. Dabei wird das Bett durch das hindurchgeleitete Abwasser und/oder Abgas aufgelockert und gewissermaßen zum Schweben gebracht. Soll die Erfindung zur Abwasserreinigung eingesetzt werden, so wird besonders das sogenannte Wirbelbettprinzip bevorzugt. Demnach werden die Reaktionsteilchen in einem Wirbelbettreaktor mit dem Abwasser voll durchgemischt. Auf diese Weise wird ein besonders effektiver Stoffaustausch zwischen den Abwasserinhaltsstoffen und den Reaktionsteilchen und ein, von der Strömung her gesehen, problemloser Betrieb erreicht. Die Teilchen werden mittels Sieben oder Schwerkraft im Reaktor zurückgehalten.

Das Verfahren eignet sich gleichermaßen für die anaerobe wie aerobe biologische Abwasserreinigung. Bei der aeroben Abwasserreinigung ist zur Abdeckung der sehr hohen Sauerstoffzehrung eine Rein-Sauerstoff-Begasung zu empfehlen.

Das Verfahren kann insbesondere zur Nitrifikation und/oder Denitrifikation von Abwasser eingesetzt werden. Es eignet sich aber auch zum Haupt- oder Restkohlenstoffabbau. Mit dem erfindungsgemäßen Verfahren können auch Nitrifikation, Denitrifikation und Kohlenstoffabbau simultan durchgeführt werden. Das Verfahren eignet sich gleichermaßen zur Trink- und Brauchwasseraufbereitung.

Bei der biologischen Abgasreinigung wird das Abgas entweder direkt durch ein fluidisiertes Bett der Reaktionsteilchen geleitet, oder das Abgas wird zunächst einer Wäsche unterzogen und anschließend das mit den Abgasinhaltsstoffen beladene Waschwasser in einem mit den Reaktionsteilchen gefüllten Reaktor entsprechend der trägergebundenen Abwasserreinigung aufbereitet.

Wird das Verfahren bei biologischen Prozessen, wie der biologischen Abwasser- oder Abgasreinigung oder der Fermentation, eingesetzt, so bildet sich während der Anfahrphase auf der Oberfläche der Reaktionsteilchen ein reaktiver Biofilm aus. Dabei wirken die im Abwasser bzw. Abgas vorhandenen Verunreinigungen als Nährstoffe für die Mikroorganismen. Um den Anfahrprozeß zu beschleunigen oder um spezielle Abwässer bzw. Abgase zu behandeln, ist gemäß einer Weiterbildung der Erfindung vorgesehen, die Reaktionsteilchen mit speziellen Mikroorganismen zu impfen. In diesem Fall eignen sich besonders solche Reaktionsteilchen, deren Oberflächenbeschaffenheit durch die oben beschriebenen Behandlungsmethoden so verändert wurde, daß sich auch sehr langsam wachsende Mikroorganismen, die für den Abbau außergewöhnlicher Abwasser- oder Abgasinhaltsstoffe geeignet sind, ansiedeln können.

Da die erfindungsgemäß verwendeten Reaktionsteilchen eine sehr viel größere spezifische Oberfläche besitzen als die herkömmlicherweise bei der trägergebundenen biologischen Abwasser- und/oder Abgasreinigung eingesetzten Trägerteilchen, kommt man mit einem geringeren Füllgrad des Reaktors aus, um diesselbe Reinigungsleistung zu erzielen. Etwa 3 Vol% der erfindungsgemäßen Reaktionsteilchen mit einer Dicke von 0,2 mm ergeben eine reaktorspezifische Oberfläche von 300 m²/m³ Reaktor. Vorzugsweise wird die Reaktorfüllung so eingestellt, daß das Verhältnis von untergebrachter Bewuchsfläche zu Reaktorvolumen ca. 50 m²/m³ bis ca. 2000 m²/m³ beträgt.

Das erfindungsgemäße Verfahren kann zur Durchführung der verschiedensten Reaktionen bei der Abwasser-, Trinkwasser- oder Abgasreinigung eingesetzt werden. Dabei können die Reaktionsteilchen selbst aus einem reaktiven Material gefertigt sein, die bestimmte Reaktionen einleiten oder bewirken, z.B. kann durch Aluminiumfolien oder PHB-Folien Nitrat aus dem Trinkwasser entfernt werden. Andererseits können die Reaktionsteilchen mit Reaktiv-stoffen (z.B. Eisen) dotiert werden, die ihrerseits die Reaktionen in Gang setzen. Als besonders vielversprechend ist der Einsatz der Reaktionsteilchen bei der trägerge-bundenen Abwasser- und/oder Abgasreinigung anzusehen. Auf den Reaktionsteilchen siedelt sich dabei ein besonders aktiver Biofilm an, so daß hohe Reinigungsleistungen erzielt werden können.

Die Erfindung bietet wesentliche Vorteile gegenüber dem Stand der Technik. Die hohe spezifische Oberfläche der Reaktionsteilchen erlaubt beispielsweise in einem Abwasserreaktor eine sehr hohe Reinigungsleistung pro Reaktorvolumen. Die kleinen, leichten Reaktionsteilchen schweben im Wasser und können mit äußerst geringem Energieaufwand gepumpt oder im Reaktor bewegt werden. Das Material für die Reaktionsteilchen kann im Hinblick auf das gewünschte spezifische Gewicht, die Haltbarkeit und die physiologische Unbedenklichkeit frei gewählt werden. Außerdem ist das erfindungsgemäße Verfahren äußerst wirtschaftlich, da die Reaktionsteilchen billig herzustellen sind. Beispielsweise können die Reaktionsteilchen aus Kunststoffabfällen oder Recyclingmaterial gefertigt werden. Im folgenden wird die Erfindung anhand von in den Figuren schematisch dargestellten Ausführungsbeispielen näher erläutert:

Es zeigen:
- Figur 1:: ein Fließschema eines Verfahrens zur biologischen Abwasserreinigung mit den Reaktionsteilchen als Aufwuchsfläche für Biomasse
- Figur 2:: eine Skizze eines Reaktionsteilchens in eckiger und runder Form
- Figur 3:: ein Diagramm zur Abhängigkeit der spezifischen Oberfläche der Reaktionsteilchen von der Dicke der Reaktionsteilchen
- Figur 4:: einen Querschnitt eines aus einer Verbundfolie gefertigten Reaktionsteilchens
- Figur 5:: einen Querschnitt eines Reaktionsteilchens mit eingepreßtem Substrat.
- Figur 6:: einen Querschnitt eines Reaktionsteilchens mit Dellen auf der Oberfläche.

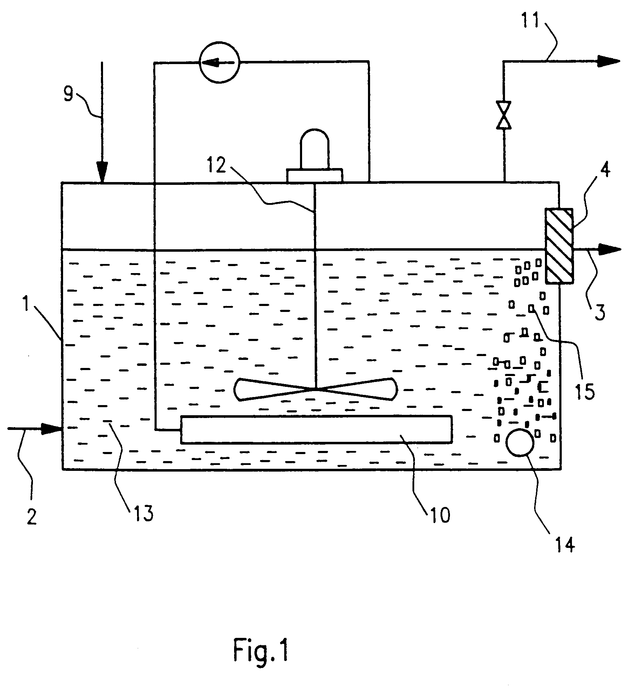

In Figur 1 ist mit 1 ein gegen die Atmosphäre geschlossener und als volldurchmischtes Belebungsbecken ausgebildeter Reaktor bezeichnet. In dem Belebungsbecken 1 sind, wie durch die Schraffierung 13 angedeutet, als Trägermaterial für die Mikroorgansimen planare Teilchen mit einer Dicke von ca. 200 µm bis ca. 500 µm und einer Fläche von ca. 100 (mm)² bis ca. 500 (mm)² in einer Menge freibeweglich angeordnet, die einer untergebrachten Bewuchsfläche von ca. 500 bis ca. 1000 m²/m³ Reaktorvolumen entspricht. Die Reaktionsteilchen bestehen aus einer im ursprünglichen Zustand ca. 30 µm dicken, perforierten Polypropylenfolie mit einer Lochdichte von ca. 200 Löcher/cm². Die Löcher weisen einen Durchmesser von ca. 200 bis ca. 600 µm auf. Die Folie wurde einer Oberflächenbehandlung, einer sogenannten Korona-Behandlung unterzogen. Das dabei entstehende Ozon greift die Matrix der Folie an und schafft freie Valenzen, wodurch die elektrische Oberflächenbeschaffenheit verändert wird. Auf der so behandelten Folie können sich die Mikroorganismen besonders schnell ansiedeln.

Das zu behandelnde Abwasser wird über einen Zulauf 2 in das Belebungsbecken 1 eingeleitet, während das behandelte Abwasser über einen im oberen Bereich des Belebungsbeckens 1 angeschlossenen Ablauf 3, dem eine Trenneinrichtung 4 zum Zurückhalten der einzelnen Stoffteilchen, die beispielsweise ein einfaches Sieb sein kann, abgezogen wird. Zur Sauerstoffversorgung der Mikroorganismen ist vorgesehen, dem sich unter der Abdeckung des Belebungsbeckens 2 ausbildenden Gasraum über eine Zufuhrleitung 9 technisch reinen Sauerstoff oder zumindest ein mehr Sauerstoff als Luft enthaltendes Gas zuzuleiten und das im Gasraum befindliche Gas über eine Leitung einem nahe dem Boden des Belebungsbeckens 1 angeordneten Gasverteiler 10 zuzuführen. Abgas wird über eine ventilgesteuerte Abgasleitung 11 abgezogen. Die aufsteigenden Gasblasen des Belüftungsgases erzeugen dabei genügend Auftrieb, um die mit Biomasse beladenen Reaktionsteilchen 13, deren spezifisches Gewicht im bewachsenen Zustand etwa dem von Wasser entspricht, in Bewegung zu versetzen.

Um eine möglichst gute Durchmischung und einen guten Stoffumsatz zu erreichen, ist eine Umwälzeinrichtung 12, beipielsweise bestehend aus einer einfachen Rühreinrichtung mit Elektromotorantrieb, unmittelbar über dem Gasverteiler 10 angeordnet

Neben der in der Figur gezeigten Anordnung der Zufuhrleitung 9 für Behandlungsgas besteht auch die Möglichkeit, eine Gaszufuhrleitung direkt über mehrere Anschlüsse am Boden des Belebungsbeckens 1 anzuschließen und auf diese Weise eine zusätzliche, nach oben gerichtete Gasströmung zu erzeugen. Ebenso kann der Abwasserzulauf 2 mehrere über dem Beckenboden verteilte Anschlüsse aufweisen, um im Belebungsbecken auch eine nach oben gerichtete Flüssigkeitsströmung aufrecht zu erhalten.

Zum Freihalten der Trenneinrichtung 4 ist eine unmittelbar vor der Trenneinrichtung 4 angeordnete Tiefenbelüftungseinrichtung 14 vorgesehen, die eine nach oben gerichtete hohe Strömungsgeschwindigkeit von ca. 0,5 m/s erzeugt. Außerdem werden sphärische Putzkörper 15, insbesondere Schaumstoffwürfel, in einer Menge von ca. 1 bis 10 Vol% dem Reaktor 1 zugegeben. Die mit der Abwasserströmung nach oben mitgerissenen Putzkörper 15 reiben an der Trenneinrichtung 4 und reinigen diese von abgesetzten Feststoffen oder Reaktionsteilchen. Die kleinen und leichten Reaktionsteilchen lassen sich mit äußerst geringem Energieaufwand pumpen.

In der nachstehenden Tabelle sind Zahlenangaben für ein Auslegungsbeispiel einer nach dem erfindungsgemäßen Verfahren betriebenen Nitrifikationsanlage im Vergleich zu einer herkömmlichen Trägermaterialanlage angegeben.

| | **Trägergebundene Verfahren** | |
|---|---|---|
| | herkömmliche Trägerverfahren | mit den erfindungsgemäßen Reaktionsteilchen als Trägerteilchen |
| Abwasserzulaufmange (m³/d) | 20.000 | 20.000 |
| NH⁺₄-N-Zulauf [mg N/l] | 50 | 50 |
| NH₄N-Ablauf [mg N/l] | < 10 | < 1 |
| Zulässige Stickstoff-Flächenbelastung [g N/m² d] | 2 | 2,5 |
| zu installierende Biofläche [m^{2]} | 400.000 | 400.000 |
| erforderliche Folienfläche [m²] | - | 200.000 |
| Folienmaterial | - | Polypropylen Koronabehandelt |
| Foliendicke (ursprünglich) [µm] | - | 30 |
| Foliendicke gelocht [µm] | - | 300 |
| Teilchengröße [mm²] | - | ca. 200 |
| Loch-Durchmesser [µm] | - | 200..400 |
| Lochdichte [Lö/cm²] | - | 100..200 |
| Spezifische Oberfläche bezogen auf Kompaktmaterial [m²/m³] | 250 | ca. 7000 |
| Spezifische Oberfläche bezogen auf Reaktorvolumen [m²m³R] | 175 | 525 |
| Reaktorvolumen [m³] | 2500 | 800 |
| Kosten für Trägermaterial [Mio DM] | 0,6 | 0,1 |
| Gesamtinvestitionskosten für den Reaktor [Mio DM] | 1,85 | 0,6 |

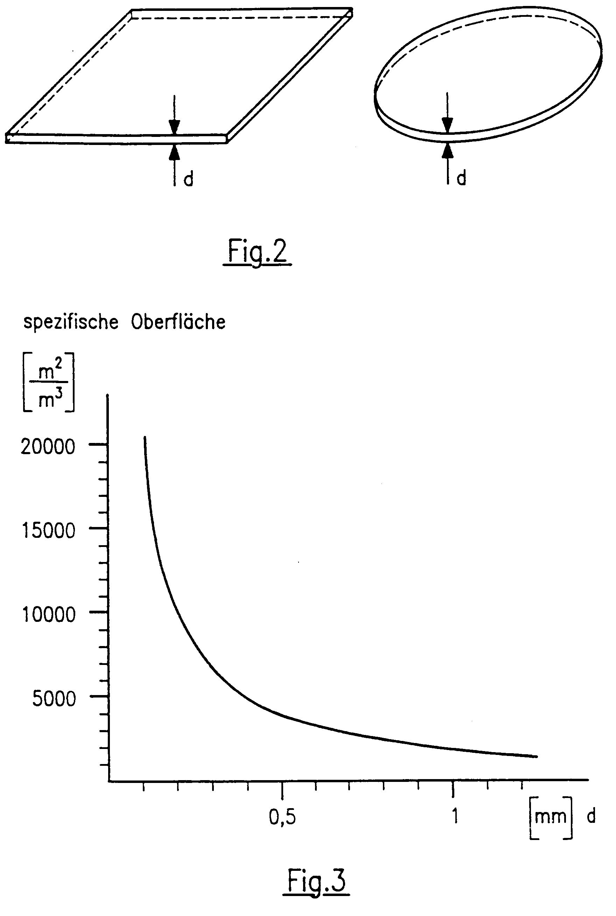

Figur 2 zeigt eine Skizze eines erfindungsgemäßen Reaktionsteilchens in eckiger und runder Form. Mit d ist die Dicke des Reaktionsteilchens bezeichnet.

Wie aus dem Diagramm von Figur 3 hervorgeht, nimmt die spezifische Oberfläche der Reaktionsteilchen mit der Dicke d der Reaktionsteilchen exponentiell ab. Die spezifische Oberfläche ist umso größer, je kleiner die Dicke der Reaktionsteilchen ist. Ein günstiger Bereich für die spezifische Oberfläche (4000 bis über 20 000 m²/m³) ergibt sich bei Dicken von d = 0,01 mm bis d = 0,5 mm. Zum Vergleich sind nachfolgend typische Werte für spezifische Oberflächen von herkömmlichen Trägerteilchen angegeben:

| | |
|---|---|
| Sand (Durchmesser = 0,7 mm) | 850 m²/m³ |
| biologisch aktive Schaumwürfel (Kantenlänge 1 cm) | 1000 m²/m³ |
| Tropfkörpermaterial | 100-300 m²/m³ |

Die erfindungsgemäßen Reaktionsteilchen weisen also eine um ein Vielfaches größere spezifische Oberfläche als herkömmliche Trägerteilchen auf.

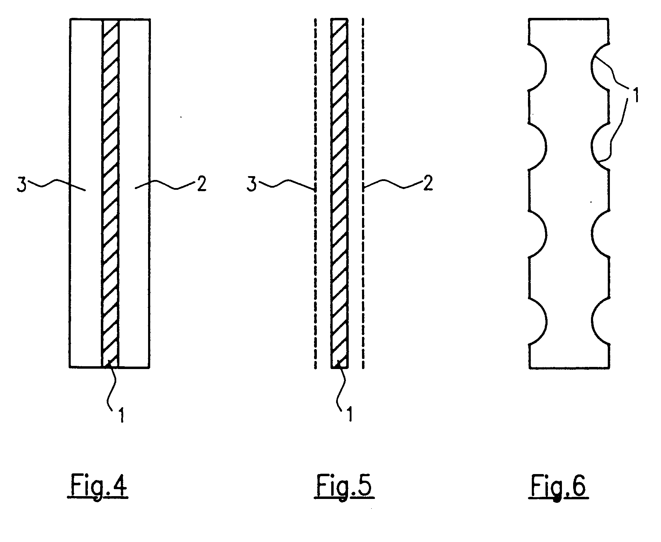

In Figur 4 ist ein Reaktionsteilchen im Querschnitt dargestellt, das aus zwei verschiedenen Folien in Sandwich-Bauweise zusammengesetzt ist. Die innere Schicht 1 bildet eine Aluminiumfolie mit einer Dichte von 2,7 g/cm³. Die beiden äußeren Schichten 2 und 3 werden von Polyethylenfolien mit einer Dichte von 0,9 g/cm³ gebildet. Wenn z.B. die Polyethylenfolien insgesamt einen Anteil von 6/7 und die Aluminiumfolie einen Anteil von 1/7 an der gesamten Dicke des Reaktionsteilchens ausmachen, so ergibt sich eine mittlere Dichte von 1,15 g/cm³. Auf diese Weise kann durch entsprechende Wahl der einzelnen Schichtmaterialien und Schichtdicken prinzipiell jede gewünschte Dichte des Reaktionsteilchens eingestellt werden.

Figur 5 zeigt einen Querschnitt eines Reaktionsteilchens, das aus zwei äußeren Membranfolien 2 und 3 und einer inneren Substratfolie 1 zusammengesetzt ist. Die Substratfolie, z.B. Polyhydroxibuttersäure, kann durch die Membranfolie hindurch z.B. in Wechselwirkung mit den Abwasser- und/oder Abgasinhaltsstoffen treten.

In Figur 6 ist ein Querschnitt eines Reaktionsteilchens dargestellt, das Dellen 1 auf der Oberfläche aufweist. Die Dellen 1 haben vorzugsweise einen Durchmesser von ca. 0,05 bis ca. 0,5 und eine Tiefe von bis zu 0,5 mm.

## Patentansprüche

1. Verfahren zur Reinigung von Trinkwasser oder Abwasser und/oder Abgas, wobei das Trinkwasser oder Abwasser und/oder Abgas mit Reaktionsteilchen in Kontakt gebracht wird, die die Reinigung fördern oder ermöglichen, **dadurch gekennzeichnet**, daß als Reaktionsteilchen im wesentlichen planare Teilchen mit einer Dicke von ca. 5 µm bis ca. 1500 µm und einer Fläche von ca. 5 (mm)² bis ca. 1000 (mm)² verwendet werden, die in einem Reaktor zum Schweben gebracht und/oder in Bewegung versetzt werden.

2. Verfahren nach Anspruch 1, dadurch gekennzeichnet, daß das Abwasser und/oder Abgas durch ein fluidisiertes Bett der Reaktionsteilchen hindurchgeleitet wird.

3. Verfahren nach Anspruch 1, dadurch gekennzeichnet, daß das Abwasser in einem Wirbelbettreaktor mit den Reaktionsteilchen voll durchmischt wird.

4. Verfahren nach einem der Ansprüche 1 bis 3, dadurch gekennzeichnet, daß auf die Reaktionsteilchen Biomasse aufgebracht wird, oder ein spontanes Aufwachsen von Biomasse ermöglicht wird.

5. Verfahren nach einem der Ansprüche 1 bis 4, dadurch gekennzeichnet, daß Reaktionsteilchen mit einer Dicke von ca. 15 µm bis ca. 500 µm verwendet werden.

6. Verfahren nach einem der Ansprüche 1 bis 5, dadurch gekennzeichnet, daß als Reaktionsteilchen Teilchen verwendet werden, die außerdem Löcher mit einem Durchmesser von ca. 10 µm bis ca. 1000 µm aufweisen.

7. Verfahren nach Anspruch 6, dadurch gekennzeichnet, daß als Reaktionsteilchen Teilchen verwendet werden, die ca. 10 bis ca. 1000 Löcher/cm² aufweisen.

8. Verfahren nach einem der Ansprüche 1 bis 7, dadurch gekennzeichnet, daß als Reaktionsteilchen Teilchen verwendet werden, die Prägungen auf der Teilchenoberfläche aufweisen.

9. Verfahren nach einem der Anprüche 1 bis 8, dadurch gekennzeichnet, daß als Reaktionsteilchen Teilchen verwendet werden, auf deren Oberfläche Fasern und/oder Pulver aufgebracht sind.

10. Verfahren nach einem der Ansprüche 1 bis 8, dadurch gekennzeichnet, daß als Reaktionsteilchen Teilchen verwendet werden, auf deren Oberfläche ein Vlies aufgebracht ist.

11. Verfahren nach einem der Ansprüche 1 bis 10, dadurch gekennzeichnet, daß als Reaktionsteilchen Teilchen verwendet werden, deren Oberfläche mit Reaktionsstoffen, insbesondere Zeolithpulver, Aktivkohle oder Eisen, dotiert ist.

12. Verfahren nach einem der Ansprüche 1 bis 11, dadurch gekennzeichnet, daß als Reaktionsteilchen Teilchen verwendet werden, die aus Kunststoffgewebe bestehen.

13. Verfahren nach einem der Ansprüche 1 bis 12, dadurch gekennzeichnet, daß als Reaktionsteilchen Teilchen verwendet werden, die aus Polyolefinen bestehen.

14. Verfahren nach einem der Ansprüche 1 bis 12, dadurch gekennzeichnet, daß als Reaktionsteilchen Teilchen verwendet werden, die aus Polystyrol bestehen.

15. Verfahren nach einem der Ansprüche 1 bis 12, dadurch gekennzeichnet, daß als Reaktionsteilchen Teilchen verwendet werden, die aus biologisch leicht abbaubaren Bio-Kunststoffen bestehen.

16. Verfahren nach einem der Ansprüche 1 bis 11, dadurch gekennzeichnet, daß als Reaktionsteilchen Teilchen verwendet werden, die aus Metallfolien bestehen.

17. Verfahren nach einem der Ansprüche 1 bis 15, dadurch gekennzeichnet, daß als Reaktionsteilchen Teilchen aus Kunststoff verwendet werden, die ein spezifisches Gewicht von ca. 0,8 g/cm³ bis ca. 1,5 g/cm³ aufweisen.

## Claims

1. Method of purifying drinking water or waste water and/or offgas, in which the drinking water or waste water and/or offgas is brought into contact with reaction particles which promote or make possible the purification, characterized in that, as reaction particles, use is made of essentially planar particles which have a thickness of approximately 5 µm to approximately 1500 µm and an area of approximately 5 (mm)² to approximately 1000 (mm)² and which are suspended and/or set in motion in a reactor.

2. Method according to Claim 1, characterized in that the waste water and/or offgas is passed through a fluidized bed of the reaction particles.

3. Method according to Claim 1, characterized in that the waste water is completely mixed with the reaction particles in a fluidized bed.

4. Method according to one of Claims 1 to 3, characterized in that biomass is applied to the reaction particles or a spontaneous overgrowth of biomass is made possible.

5. Method according to one of Claims 1 to 4, characterized in that reaction particles having a thickness of approximately 15 µm to approximately 500 µm are used.

6. Method according to one of Claims 1 to 5, characterized in that, as reaction particles, use is made of particles which have, in addition, holes having a diameter of approximately 10 µm to approximately 1000 µm.

7. Method according to Claim 6, characterized in that, as reaction particles, use is made of particles which have approximately 10 to 1000 holes/cm².

8. Method according to one of Claims 1 to 7, characterized in that, as reaction particles, use is made of particles which have impressions on the particle surface.

9. Method according to one of Claims 1 to 8, characterized in that, as reaction particles, use is made of particles to whose surface fibres and/or powders have been applied.

10. Method according to one of Claims 1 to 8, characterized in that, as reaction particles, use is made of particles to whose surface a fibrous web has been applied.

11. Method according to one of Claims 1 to 10, characterized in that, as reaction particles, use is made of particles whose surface has been doped with reactive substances, in particular zeolite powder, active carbon or iron.

12. Method according to one of Claims 1 to 11, characterized in that, as reaction particles, use is made of particles which are composed of plastic fabric.

13. Method according to one of Claims 1 to 12, characterized in that, as reaction particles, use is made of particles which are composed of polyolefins.

14. Method according to one of Claims 1 to 12, characterized in that, as reaction particles, use is made of particles which are composed of polystyrene.

15. Method according to one of Claims 1 to 12, characterized in that, as reaction particles, use is made of particles which are composed of easily biodegradable biological plastics.

16. Method according to one of Claims 1 to 11, characterized in that, as reaction particles, use is made of particles which are composed of metal foils.

17. Method according to one of Claims 1 to 15, characterized in that, as reaction particles, use is made of plastic particles which have a density of approximately 0.8 g/cm³ to approximately 1.5 g/cm³.

## Revendications

1. Procédé pour la purification d'eau potable ou d'eaux usées et/ou d'effluents gazeux, dans lequel l'eau potable ou les eaux usées et/ou les effluents gazeux sont amenés en contact avec des particules de réaction, qui facilitent ou rendent possible la purification, caractérisé en ce qu'on utilise comme particules de réaction des particules sensiblement planes d'une épaisseur d'environ 5 µm à environ 1500 µm et d'une superficie d'environ 5 (mm)² à environ 1000 (mm)², qui sont amenées en suspension et/ou mises en mouvement dans un réacteur.

2. Procédé suivant la revendication 1, caractérisé en ce qu'on fait passer les eaux usées et/ou les effluents gazeux à travers un lit fluidisé des particules de réaction.

3. Procédé suivant la revendication 1, caractérisé en ce que les eaux usées sont complètement mélangées dans un réacteur à lit fluidisé avec les particules de réaction.

4. Procédé suivant l'une quelconque des revendications 1 à 3, caractérisé en ce que de la biomasse est déposée sur les particules de réaction, ou qu'une croissance spontanée de biomasse est rendue possible.

5. Procédé suivant l'une quelconque des revendications 1 à 4, caractérisé en ce qu'on utilise des particules de réaction d'une épaisseur d'environ 15 µm à environ 500 µm.

6. Procédé suivant l'une quelconque des revendications 1 à 5, caractérisé en ce qu'on utilise comme particules de réaction des particules qui présentent en outre des perforations d'un diamètre d'environ 10 µm à environ 1000 µm.

7. Procédé suivant la revendication 6, caractérisé en ce qu'on utilise comme particules de réaction des particules qui présentent environ 10 à environ 1000 perforations/cm².

8. Procédé suivant l'une quelconque des revendications 1 à 7, caractérisé en ce qu'on utilise comme particules de réaction des particules qui présentent des alvéoles sur la surface de particule.

9. Procédé suivant l'une quelconque des revendications 1 à 8, caractérisé en ce qu'on utilise comme particules de réaction des particules sur la surface desquelles sont déposées des fibres et/ou des poudres.

10. Procédé suivant l'une quelconque des revendications 1 à 8, caractérisé en ce qu'on utilise comme particules de réaction des particules sur la surface desquelles est déposé un mat de fibres.

11. Procédé suivant l'une quelconque des revendications 1 à 10, caractérisé en ce qu'on utilise comme particules de réaction des particules dont la surface est dopée au moyen de matières de réaction, en particulier de la poudre de zéolite, du charbon actif ou du fer.

12. Procédé suivant l'une quelconque des revendications 1 à 11, caractérisé en ce qu'on utilise comme particules de réaction des particules qui sont constituées de tissu en matière synthétique.

13. Procédé suivant l'une quelconque des revendications 1 à 12, caractérisé en ce qu'on utilise comme particules de réaction des particules qui sont constituées de polyoléfines.

14. Procédé suivant l'une quelconque des revendications 1 à 12, caractérisé en ce qu'on utilise comme particules de réaction dos particules qui sont constituées de polystyrène.

15. Procédé suivant l'une quelconque des revendications 1 à 12, caractérisé en ce qu'on utilise comme particules de réaction des particules qui sont constituées de matière plastique facilement biodégradables.

16. Procédé suivant l'une quelconque des revendications 1 à 11, caractérisé en ce qu'on utilise comme particules de réaction des particules qui sont constituées de feuilles métalliques minces.

17. Procédé suivant l'une quelconque des revendications 1 à 15, caractérisé en ce qu'on utilise comme particules de réaction des particules en matière plastique qui présentent une masse spécifique d'environ 0,8 g/cm³ à environ 1,5 g/cm³.
